(19) Europäisches Patentamt / European Patent Office / Office européen des brevets

(11) **EP 1 840 564 B1**

(12) **EUROPEAN PATENT SPECIFICATION**

(45) Date of publication and mention
of the grant of the patent:
**20.09.2017 Bulletin 2017/38**

(51) Int Cl.:
*G01N 29/07* *(2006.01)*          *G01N 29/38* *(2006.01)*
*G01N 29/44* *(2006.01)*

(21) Application number: **07005992.8**

(22) Date of filing: **23.03.2007**

(54) **Digital beamforming apparatus with a sigma-delta A/D converter**

Digitale Strahlformungsvorrichtung mit einem Sigma-Delta-A/D-Wandler

Appareil de formation digitale de faisceaux avec convertisseur analogique/numérique sigma-delta

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **29.03.2006 KR 20060028339**

(43) Date of publication of application:
**03.10.2007 Bulletin 2007/40**

(73) Proprietor: **Samsung Medison Co., Ltd.
Hongcheon-gun, Gangwon-do 25108 (KR)**

(72) Inventors:
• **Bae, Moo Ho
Seoul 138-240 (KR)**
• **Kim, Chang Sun
Gwangju-si
Gyeonggi-do 464-760 (KR)**
• **Kim, Yung Gil
Seoul 135-280 (KR)**

(74) Representative: **Lorenz, Markus
Lorenz & Kollegen
Patentanwälte Partnerschaftsgesellschaft mbB
Alte Ulmer Straße 2
89522 Heidenheim (DE)**

(56) References cited:
**WO-A2-00/10638          US-A1- 2004 189 499
US-B1- 6 400 295          US-B1- 6 970 126**

• **SONG T K ET AL: "A new digital phased array
system for dynamic focusing and steering with
reduced sampling rate", ULTRASONIC IMAGING,
DYNAMEDIA INC., SILVER SPRING, MD, US, vol.
12, no. 1, 1 January 1990 (1990-01-01), pages 1-16,
XP023046626, ISSN: 0161-7346, DOI:
10.1016/0161-7346(90)90217-L [retrieved on
1990-01-01]**

**Description**

**BACKGROUND**

1. **Field**

The present invention generally relates to an ultrasound system, and more particularly to a digital beamforming apparatus adopting a sigma-delta analog-to-digital (A/D) converter in the ultrasound system.

2. **Background**

An ultrasound system has become an important and popular diagnostic tool since it has a wide range of applications. Specifically, due to its non-invasive and nondestructive nature, the ultrasound system has been extensively used in the medical profession. Modern high-performance ultrasound systems and techniques are commonly used to produce two or three-dimensional diagnostic images of internal features of an object (e.g., human organs). In order to accurately examine the internal features, resolution of the ultrasound image is one of the important factors for the ultrasound system. Recently, an array transducer and transmit/receive-focusing techniques have been adopted to achieve high resolution of the ultrasound image. The array transducer includes a plurality of transducer elements, which are linearly or 2-dimensionally arrayed.

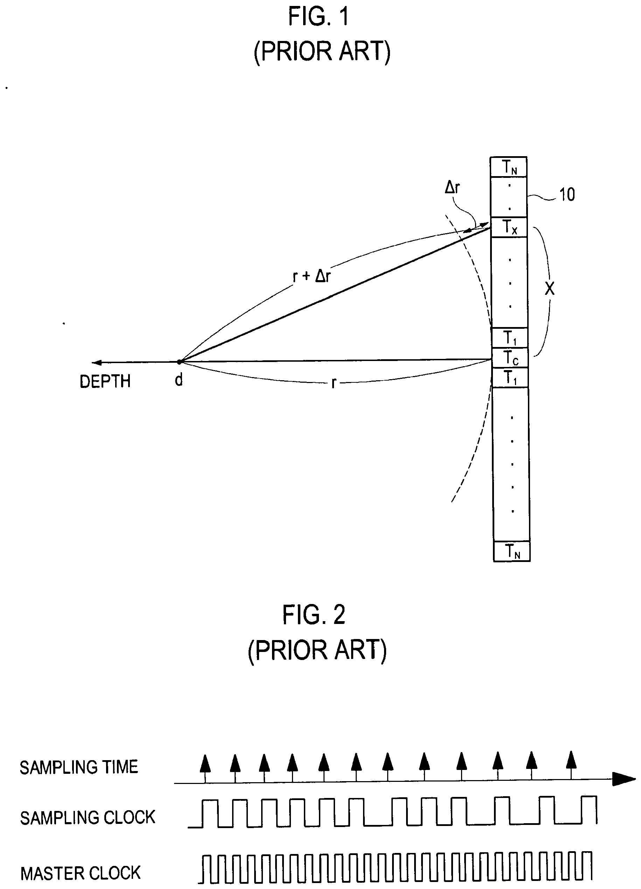

FIG. 1 is a schematic diagram illustrating the delays in ultrasound echo signals arriving at each element in an array transducer. A predetermined delay profile of transmit pulse signals is established so that ultrasound signals produced at the array transducer 10 in response to the transmit pulse signals are focused on a focal point. Each element then produces ultrasound signals according to such predetermined delay profile. Ultrasound echo signals reflected from the focal point arrive at each transducer element in different times.

As shown in FIG. 1, an ultrasound echo signal reflected from the focal point d travels a distance of "r" to arrive at a transducer element Tc. Further, an ultrasound echo signal reflected from the focal point travels a distance of r+∆r to reach a transducer element Tx. That is, the ultrasound echo signal received at the transducer element Tx is delayed by ∆r compared to the ultrasound echo signal received at the transducer element Tc. The ultrasound echo signals received at each transducer element are converted into electrical signals (hereinafter referred to as receive signals). The receive signals should be focused in order to obtain image signals. In focusing the receive signals, the delays in the ultrasound echo signals arriving at each transducer element should be compensated. A receive focusing delay technique is usually adopted to compensate for the delays in the ultrasound echo signals.

Conventionally, the delays in the ultrasound echo signals are compensated by using L/C passive delay devices at each channel in a beamformer of the ultrasound system and the compensated signals are finally focused in an adder. The receive signals delayed by the L/C passive delay devices are focused and then sampled to obtain ultrasound image data. In such a case, a significant number of passive delay devices are needed to reduce compensation errors, which causes problems in that the ultrasound system becomes large and complicated.

In order to reduce the complexity of the beamformer using the passive delay devices in the ultrasound system, a dynamic receive focusing technique such as a sample-delay focusing (SDF) technique has been introduced, rather than using the passive delay devices. The SDF technique is carried out by sampling and delaying the receive signals at the same time, and then focusing the sampled signals.

Further, as the beamformer of the ultrasound system has been developed into a digital beamformer, an analog-to-digital (A/D) converter should be installed at each channel of the digital beamformer. Therefore, the A/D converter with a simple structure is required to reduce the complexity of the digital beamformer. In this respect, a sigma-delta A/D converter has received great attention due to its simple structure and ability to control a finite delay at low power.

Recently, there has been an attempt to apply the SDF technique to the sigma-delta A/D converter in order to simplify the structure of the digital beamformer of the ultrasound system. In the SDF technique, a cycle of a sampling clock signal, which is generated in synchronization with a master clock provided in the ultrasound system, must be adjusted. This is because a sampling interval is increased as a distance between the array transducer and the focal point becomes longer. That is, the cycle of the sampling clock signal may be changed depending on the location, as shown in FIG. 2. In such a case, the sampling may not be accurately carried out since the sampling time is not matched with an edge of the sampling clock signal. As such, the sigma-delta A/D converter cannot output a correct digital value.

From WO00/10638 A2 there is known an asynchronous oversampling beamformer using a set of delta-sigma (DELTA SIGMA) analog-to-digital (A/D) converters without affecting the image quality. The echoes reflected from the region of interest are sensed by plurality of transducers which comprises the transducer array. The signals received by the transducer array are sampled at the time instants required for the receive beamforming using the timing information stored in a memory and then digitized by DELTA SIGMA modulators prior to summation.

## BRIEF DESCRIPTION OF THE DRAWINGS

Arrangements and embodiments may be described in detail with reference to the following drawings in which like reference numerals refer to like elements and wherein:

FIG. 1 is a schematic diagram illustrating the delays in ultrasound echo signals arriving at each transducer element in an array transducer;

FIG. 2 is a diagram showing a sampling clock generated in synchronization with a master clock;

FIG. 3 is a schematic diagram showing an example of a digital receive-focusing device using a sigma-delta analog-to-digital (A/D) converter;

FIG. 4 is a block diagram showing a variable sampling clock generating unit;

FIG. 5 is a block diagram showing a sigma-delta A/D converter in accordance with one embodiment of the present invention;

FIG. 6 is a circuit diagram illustrating an integrator in the sigma-delta A/D converter in accordance with one embodiment of the present invention;

FIG. 7 is a diagram showing waveforms of a pair of sampling clock signals in accordance with one embodiment of the present invention;

FIG. 8 is a circuit diagram showing an equivalent circuit of the integrator in accordance with one embodiment of the present invention;

FIG. 9 is a circuit diagram showing an example of a variable capacitor in the integrator in accordance with one embodiment of the present invention; and

FIG. 10 is a block diagram illustrating a process of generating control signals in the sampling clock generator in accordance with one embodiment of the present invention.

## DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

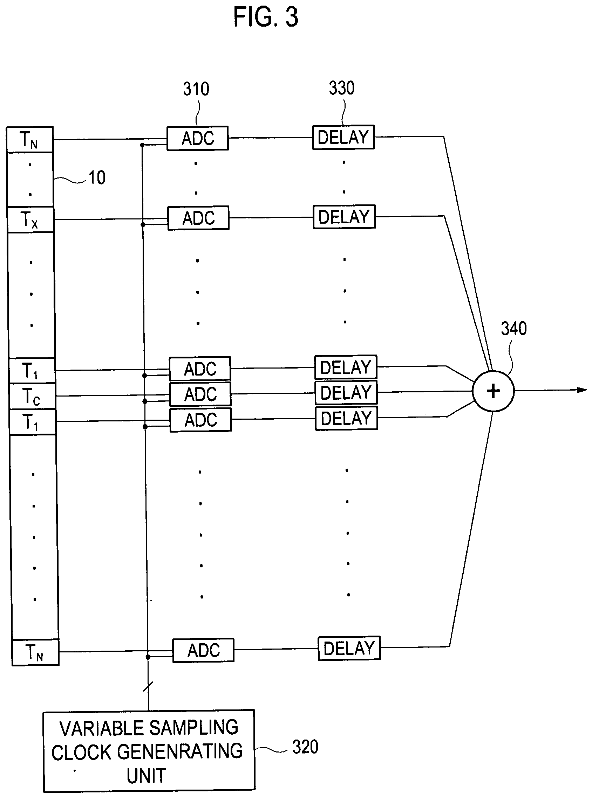

FIG. 3 is a schematic diagram showing an example of a digital beamformer adopting a sigma-delta analog-to-digital (A/D) converter. As shown in FIG. 3, the digital beamformer 300 includes a plurality of sigma-delta A/D converters 310, a variable sampling clock generating unit 320, a plurality of delay units 330 and an adder 340.

The sigma-delta A/D converters 310 operate in response to sampling the clock signals provided from the variable sampling clock generating unit 320. Each of the sigma-delta A/D converters 310 samples analog signals outputted from each element of the array transducer 110. The signal A/D converters 310 convert the sampled analog signals into digital signals.

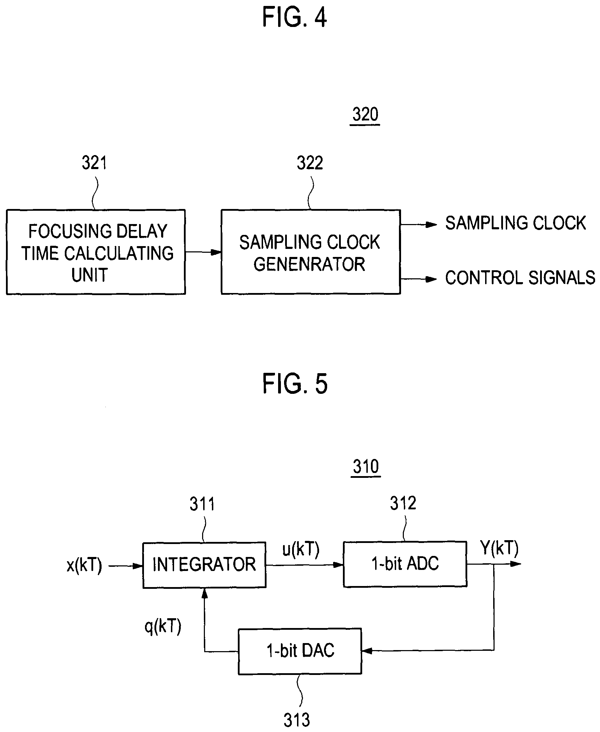

The variable sampling clock generating unit 320 provides the sampling clock signals to each signal-delta A/D converter 320 by considering the delay times, with which the ultrasound echo signals reflected from a focal point in a target object arrive at each element of the array transducer 110. As shown in FIG. 4, the variable sampling clock generating unit 320 includes a focusing delay time calculating unit 321 and a sampling clock generator 322.

The focusing delay time calculating unit 321 calculates focusing delay times for the ultrasound echo signals received at each element of the array transducer 110. The calculation of the focusing delay time may be performed based on the midpoint algorithm disclosed in U.S. Patent No. 5,836,881 entitled "FOCUSING DELAY CALCULATION METHOD FOR REAL-TIME DIGITAL FOCUSING AND APPARATUS ADOPTING THE SAME." Also, the focusing delay time may be efficiently calculated by using the algorithm disclosed in U.S. Patent No. 5,669,384 entitled "REAL TIME DIGITAL RECEPTION FOCUSING METHOD AND APPARATUS ADOPTING THE SAME."

The sampling clock generator 322 generates a pair of sampling clock signals, the phases of which are not overlapped based on the focusing delay time calculated in the focusing delay time calculating unit 321. The sampling clock signals are provided to the sigma-delta A/D converter 310. The sampling clock generator 322 generates the sampling clock signals in synchronization with a master clock provided from the ultrasound diagnostic system. When such synchronized sampling clock signals are generated, the sampling clock generator 322 detects cycle changes of the sampling clock signals. The sampling clock generator 322 outputs control signals corresponding to the detected cycle changes of the sampling clock signals to the sigma-delta A/D converter 310.

The delay unit 330 delays the digital signals outputted from the sigma-delta A/D converter 310 to compensate for the delay times, with which the ultrasound signals reflected from the focal point arrive at each element of the array transducer 110. The delay unit 330 may be implemented by being used first in a first out (FIFO) memory or variable delay lines, which is well-known in the art. The amount of delay is determined according to the focusing delay time calculated in the focusing delay time calculating unit 321. The delayed digital signals are transmitted to the adder 340.

The adder 340 then adds the delayed digital signals, thereby outputting receive-focused signals.

FIG. 5 is a block diagram showing a sigma-delta A/D converter in accordance with one embodiment of the present invention. Referring to FIG. 5, the sigma-delta A/D converter 310 includes an integrator 311, an 1-bit A/D converter 312 and an 1-bit digital-to-analog (D/A) converter 313. The integrator 311 receives the analog signal x(kT) outputted from each transducer element of the array transducer 110. The integrator 311 outputs a ramping voltage u(kT) corresponding to the magnitude of the received analog signal. The 1-bit A/D converter 312 outputs an 1-bit signal of "high" or "low" y(kT) according to an output of the integrator 311. The 1-bit A/D converter 312 may include a typical comparator. The 1-bit D/A converter 313 feeds back a reference voltage +Vref or -Vref to the integrator 311 in response to the output of the 1-bit A/D converter 312. The reference voltage +Vref or -Vref outputted from the 1-bit D/A converter 313 is preferably designed so as to ensure that the output of the integrator 311 becomes 0V.

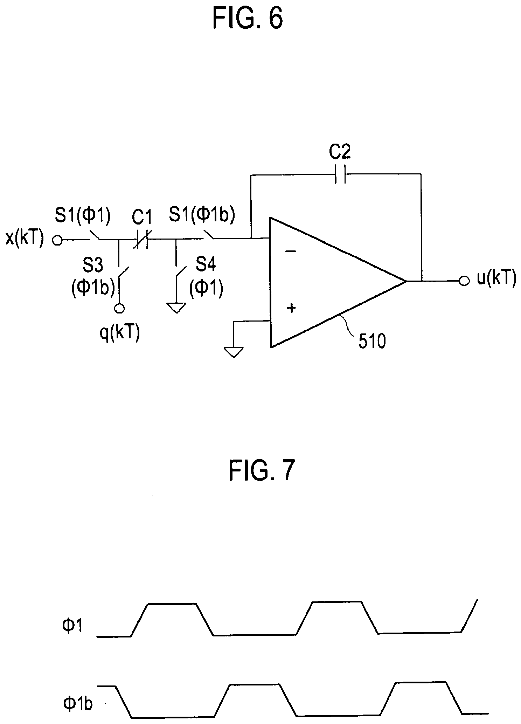

FIG. 6 is a circuit diagram illustrating the integrator in the sigma-delta A/D converter in accordance with one embodiment of the present invention. FIG. 7 shows a pair of sampling clock signals Ø1 and Ø1b, the phases of which are not overlapped and which are generated from the sampling clock generator 322. The integrator 311 includes a plurality of switches S1-S4, a variable capacitor C1, an operation amplifier (OP AMP) 510 and a capacitor C2. The plurality of switches S1-S4 become "on" or "off" in response to the sampling clock signals Ø1 and Ø1b. The capacitance of the variable capacitor C1 is variably adjusted based on the control signals transmitted from the sampling clock generator 322. A first input of the OP AMP 510 is connected to the variable capacitor C1, whereas a second input of the OP AMP 510 is connected to the ground. The capacitor C2 is coupled between the first input and an output of the OP AMP 510.

A first sampling clock 01 is applied to the first and third switches S1 and S3, while a second sampling clock Ø1b is applied to the second and fourth switches S2 and S4. If the first sampling clock 01 becomes a logic "high," then the first and third switches S1 and S3 become "on," thereby sampling the analog signals transmitted from each elements of the array transducer 110. In such a case, the second sampling clock Ø1b maintains at a logic "low" so that the second and fourth switches S2 and S4 become "off." On the other hand, if the sampling clock Ø1 becomes a logic "low," then the first and second switches S1 and S3 become an "off" state, thereby holding the sampled analog signals. In such a case, the second sampling clock becomes a logic "high" so that the second and fourth switches S2 and S4 become "on." Thus, the sampled signals added to the output signal of the 1-bit D/A converter 313 are inputted into the OP AMP 510.

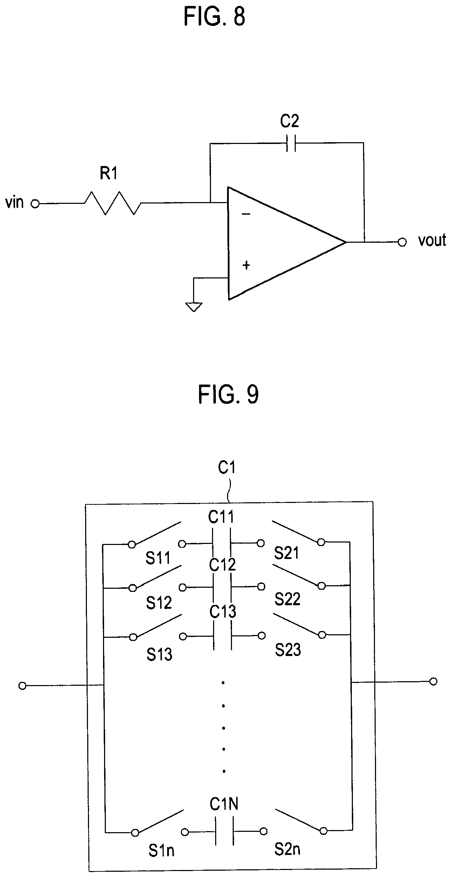

FIG. 8 is a circuit diagram showing an equivalent circuit of the integrator in accordance with one embodiment of the present invention. The capacitance of the variable capacitor C1 may become pure resistance R1 based on the switched-capacitor theory. The output of the integrator 311 depends on the time constant. The time constant T in the integrator 311 is defined by resistance R1 and capacitance C2. That is, the time constant T is defined by the following equation (1):

$$T = R1 \cdot C2 \qquad\qquad (1)$$

Also, the time constant T is in inverse proportion to the frequency of the sampling clock (T=1/F).

If the cycle of the sampling clock signal generated in the variable sampling clock generating unit 320 is changed, then the sampling clock generator 322 transmits control signals corresponding to the changed cycle of the sampling clock signal to the sigma-delta A/D converter 310. The capacitance of the variable capacitor C1 in the integrator 311 is maintained at a constant value during a normal process. However, when the cycle of the sampling clock signal is changed, the capacitance of the variable capacitor C1 is varied during one sampling cycle in response to the control signal such that the time constant T is maintained at a constant value in accordance with the present invention. For example, if the cycle of the sampling clock signal becomes shorter (i.e., the frequency of the sampling clock signal increases), then the capacitance of the variable capacitor C1 is varied to have a larger value, thereby increasing the resistance R1. On the other hand, if the cycle of the sampling clock signal becomes longer (i.e., the frequency of the sampling clock signal decreases), then the capacitance of the variable capacitor C1 is varied to have a small value, thereby decreasing the resistance R1.

FIG. 9 is a circuit diagram showing an example of the variable capacitor C1 in the integrator in accordance with one embodiment of the present invention. As shown in FIG. 9, the variable capacitor C1 includes a plurality of capacitors C11-C1N, which are arrayed in parallel. Both ends of each capacitor are connected to switches S11-S1N and S21-S2N, which operate in response to the control signals transmitted from the sampled clock generator 322. In accordance with one embodiment of the present invention, each of the capacitors C11-C1N may be configured with a unit capacitor. In such a case, the capacitance of the variable capacitor C1 is determined by the number of unit capacitors connected to the switches, which are switched "on."

Also, each of the capacitors C11-C1N may be configured to have different capacitance, which is determined according to the frequency of the sampling clock signal, in accordance with another embodiment of the present invention. In such a case, when the sampling clock signal is generated, the control signal corresponding to the frequency of the sampling clock signal is transmitted to the variable capacitor C1. The switches connected to a capacitor having capacitance corresponding to the frequency of the sampling clock signal become "on" in response to the control signals.

In accordance with one embodiment of the present invention, the control signals are digital signals and the switches S11-S1N and S21-S2N may be configured with transistors, which operate in response to the control signals.

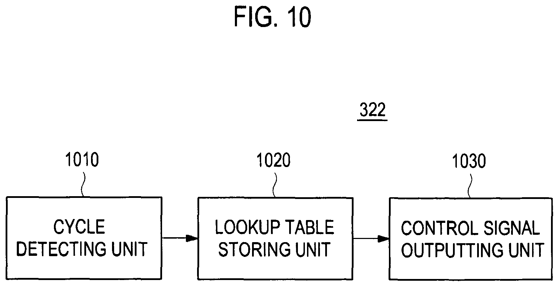

FIG. 10 is a block diagram illustrating a process of generating control signals in the sampling clock generator in accordance with one embodiment of the present invention. As shown in FIG. 10, the sampling clock generator includes a cycle detecting unit 1010, a lookup table storing unit 1020 and a control signal outputting unit 1030. The cycle detecting unit 1010 detects a cycle of the sampling clock signal and transmits a cycle detection signal corresponding to the detected cycle to the lookup table storing unit 1020. The lookup table storing unit 1020 stores a lookup table containing data relating to association of cycles of sampling clock signals with sampling times. Also, the lookup table contains information associated with sampling errors representing the time mismatches between the sampling times and edges of the sampling clock signals. The lookup table storing unit 1020 searches the lookup table in response to the cycle detection signal and outputs data corresponding to the detected cycle to the control signal outputting unit 336. The control signal outputting unit 1030 outputs digital control signals to the variable capacitor based on the data outputted from the lookup table storing unit 1020.

The cycle of the sampling clock signal is detected in the sampling clock generator in accordance with one embodiment of the present invention. Also, a cycle change detecting unit may be additionally mounted to detect a cycle of the sampling clock signal in accordance with another embodiment of the present invention.

As mentioned above, since the digital beamformer adopting the SDF technique and sigma-delta A/D converter is used in the ultrasound diagnostic system, the complexity of the system can be reduced. Also, since the time constant of the integrator included in the sigma-delta A/D converter is maintained at a constant value, the ability of the sigma-delta A/D converter can be prevented from degrading due to a cycle change of the sampling clock signal.

**Claims**

1. A digital beam forming apparatus (300) in an ultrasound system having an array transducer containing a plurality of transducer elements, comprising:

   a variable sampling clock generating unit (320) with a sampling clock generator (322) configured to variably generate sampling clock signals based on reception delay times in ultrasound echo signals arriving at each element of the transducer elements of the array transducer (110), the variable sampling clock generating unit (320) further being configured to output control signals corresponding to cycles of the sampling clock signals;
   sigma-delta analog-to-digital (A/D) converters (310) connected to the respective transducer elements of the array transducer and being configured to convert analog signals outputted from the elements of the array transducer (110) into digital signals in response to the sampling clock signals and the control signals;
   delay units (330) configured to delay output signals of the sigma-delta A/D converters based on the reception delay time; and
   an adder (340) configured to add the delayed signals,
   **characterized in that** each of the sigma-delta A/D converters (310) includes an integrator for sampling the analog signal outputted from the transducer element of the array transducer (110) in response two the sampling clock signals, the integrator having a variable capacitor whose capacitance is varied according to the control signals and an OP-Amp connected to the variable capacitor,
   wherein the capacitance of the variable capacitor is varied to have a larger value if the circle of the sampling clock signal corresponding to the control signal becomes shorter and the capacitance of the variable capacitor is varied to have a smaller value if the cycle of the sampling clock signal corresponding to the control signal becomes longer.

2. The digital beamforming apparatus of Claim 1, wherein the variable sampling clock generating unit (320) includes:

   a focusing delay time calculating unit (321) configured to calculate the delays time in the ultrasound echo signal reflected from a focal point to arrive at each transducer element of the array transducer (110)

3. The digital beamforming apparatus of Claim 2, wherein each of the sigma-delta A/D converters (310) further includes:

   an 1-bit A/D converter (312) configured to output an 1-bit digital signal in response to an output of the integrator (311); and
   an 1-bit D/A converter (313) configured to feed back a reference voltage in response to an output of the 1-bit A/D converter (312).

4. The digital beamforming apparatus (300) of Claim 1, wherein the variable capacitor includes a plurality of unit capacitors connected in parallel.

5. The digital beamforming apparatus (300) of Claim 1, wherein the variable capacitor includes a plurality of capacitors, each capacitor having a different capacitance determined according to frequencies of the sampling clock signals.

6. The digital beamforming apparatus (300) of Claim 1, wherein the variable sampling clock generator (322) includes:

a cycle detecting unit (1010) configured to detect cycles of the sampling clock signals;
a lookup table storing unit (1020) configured to store a lookup table containing data relating to association of cycles of sampling clock signals with sampling times and information associated with sampling errors representing time mismatches between the sampling times and edges of the sampling clock signals, the lookup table storing unit further being configured to search the lookup table based on the detected cycle; and
a control signal generating unit (1030) configured to output the control signals in response to the search result of the lookup table storing unit (1020).


**Patentansprüche**

1. Digitale strahlformende Vorrichtung (300) in einer Ultraschallanordnung, die einen Array-Prüfkopf hat, der eine Vielzahl von Prüfkopfelementen umfasst, aufweisend:

eine Einheit zum Erzeugen eines variablen Abgreiftaktes (320) mit einem Erzeuger zum Erzeugen des Abgreiftaktes (322), dazu ausgebildet, um variabel Abgreiftaktsignale basierend auf Empfangsverzögerungszeiten in Ultraschall-Echosignalen, die bei jedem der Element der Prüfkopfelemente des Array-Prüfkopfes ankommen, zu erzeugen, wobei die Einheit zum Erzeugen eines variablen Abgreiftaktes (320) ferner dazu ausgebildet ist, Steuersignale auszugeben, die den Zyklen der Abgreiftaktsignale entsprechen;
Sigma-Delta Analog-Digital-Konverter (A/D Konverter) (310), die mit den jeweiligen Prüfkopfelement des Array-Prüfkopfes verbunden sind und dazu ausgebildet sind, analoge Signale, die von den Elementen des Array-Prüfkopfes (110) ausgegeben werden in Antwort auf die Abgreiftaktsignale und die Steuersignale in digitale Signale umzuwandeln;
Verzögerungseinheiten (330), die dazu ausgebildet sind, die Ausgangssignale der Sigma-Delta A/D Konverter (310) basierend auf der Empfangsverzögerungszeit zu verzögern; und
einen Addierer (340), der dazu ausgebildet ist, die Verzögerungssignale hinzuzuaddieren,

**dadurch gekennzeichnet, dass** jeder der Sigma-Delta A/D Konverter (310) einen Integrator zum Abfragen der Analog Signale, die von dem Prüfkopfelement des Array-Prüfkopfes (110) ausgegeben werden in Antwort auf die Abgreiftaktsignale, wobei der Integrator einen variablen Kondensator hat, dessen Kapazität gemäß den Steuersignalen variiert wird, und einen OP-Verstärker, der mit dem variablen Kondensator verbunden ist, wobei die Kapazität des variablen Kondensators derart variiert wird, dass sie einen größeren Wert hat, wenn der Zyklus des Abgreiftaktes entsprechend dem Steuersignal kürzer wird, und die Kapazität des variablen Kondensators derart variiert wird, dass sie einen geringeren Wert hat, wenn der Zyklus des Abgreiftaktes entsprechend dem Steuersignal länger wird.

2. Digitale strahlformende Vorrichtung nach Anspruch 1, wobei die Einheit zum Erzeugen eines variablen Abgreiftaktes (320) umfasst:

eine Einheit zum Erzeugen eines fokussierten Verzögerungszeit (321), die dazu ausgebildet ist, die Verzögerungszeit in dem Ultraschall Echosignal, das von einem Fokalpunkt reflektiert wird, um an jedem Prüfkopfelement des Array-Prüfkopfes (110) anzukommen, zeitlich zu verzögern.

3. Digitale strahlformende Vorrichtung nach Anspruch 2, wobei jeder der Sigma-Delta A/D Konverter (310) ferner umfasst:

einen 1-Bit A/D Konverter (312), der dazu ausgebildet ist, ein 1-Bit Digitalsignal auszugeben in Antwort auf einen Ausgang des Integrators (311); und
einen 1-Bit D/A Konverter (313), der dazu ausgebildet ist, eine Referenzspannung zurückzuführen in Antwort auf einen Ausgang des 1-Bit A/D Konverters.

**4.** Digitale strahlformende Vorrichtung (300) nach Anspruch 1, wobei der variable Kondensator eine Vielzahl von Einheitskondensatoren umfasst, die parallel geschaltet sind.

**5.** Digitale strahlformende Vorrichtung (300) nach Anspruch 1, wobei der variable Kondensator eine Vielzahl von Einheitskondensatoren umfasst, wobei jeder Kondensator eine unterschiedliche Kapazität hat, die gemäß den Frequenzen der Abgreiftaktsignale bestimmt werden.

**6.** Digitale strahlformende Vorrichtung (300) nach Anspruch 1, wobei der Erzeuger zum Erzeugen des variablen Abgreiftaktes (322) umfasst:

eine Zyklus Ermittlungseinheit (1010), die dazu ausgebildet ist, Zyklen der Abgreiftaktsignale zu ermitteln; eine Speichereinheit zum Speichern einer Zuordnungstabelle (1020), die dazu ausgebildet ist, eine Zuordnungstabelle zu speichern, die Daten enthält, welche die Assoziation der Zyklen der Abgreiftaktsignale zu Abrufzeiten und zu Informationen, die mit Abruffehlern assoziiert werden, welche Fehlzuordnungen zwischen den Abrufzeiten und den Rändern der Abgreiftaktsignale darstellen, betreffen, wobei die Speichereinheit zum Speichern einer Zuordnungstabelle ferner dazu ausgebildet ist, die Zuordnungstabelle basierend auf dem ermittelten Zyklus zu durchsuchen; und eine Einheit zum Erzeugen eines Steuersignals (1030), die dazu ausgebildet ist, die Steuersignale in Antwort auf das Suchergebnis der Einheit zum Speichern einer Zuordnungstabelle (1020) auszugeben.

## Revendications

**1.** Appareil numérique de formation de faisceau (300) dans un système ultrasonique ayant une barrette de transducteurs constitué d'une pluralité d'éléments transducteurs, comprenant :

une unité (320) générant un échantillonnage d'horloge variable avec un générateur (322) d'échantillonnage d'horloge configuré pour générer de façon variable des signaux d'échantillonnage d'horloge basés sur la réception des délais de temporisation de signaux d'échos ultrasoniques arrivant sur chaque élément de la barrette de transducteurs (110), l'unité (320) générant un échantillonnage d'horloge variable étant en outre configuré pour émettre des signaux de commande correspondant à des cycles des signaux d'échantillonnage d'horloge ; des convertisseurs (310) analogiques-numériques (A/D) sigma-delta, connectés aux éléments transducteurs respectifs de la barrette de transducteurs et étant configuré pour convertir des signaux analogiques émis par les éléments transducteurs de la barrette de transducteurs (110), en signaux numériques en réponse aux signaux d'échantillonnage d'horloge et aux signaux de commande ; des unités de temporisation (330) configurés pour temporiser les signaux émis par les convertisseurs (310) analogiques-numériques (A/D) sigma-delta, basés sur la réception des délais de temporisation ; et un additionneur (340) configuré pour additionner les signaux temporisés, **caractérisé en ce que** chacun des convertisseurs (310) analogiques-numériques (A/D) sigma-delta, comporte un intégrateur pour échantillonner le signal analogique émis par l'élément transducteur de la barrette de transducteurs (110) en réponse aux signaux d'échantillonnage de signaux d'horloge, l'intégrateur ayant une capacité variable dont la capacitance est modifiée en fonction des signaux de commande et d'un amplificateur opérationnel connecté à la capacité variable ; dans lequel la capacitance de la capacité variable est modifiée pour avoir une valeur plus grande si le cycle de signal d'échantillonnage d'horloge correspondant au signal de commande devient plus court et la capacitance de la capacité variable est modifiée pour avoir une valeur plus petite si le cycle de signal d'échantillonnage d'horloge correspondant au signal de commande devient plus long.

**2.** Appareil numérique de formation de faisceau, selon la revendication 1, dans lequel l'unité (320) générant un échantillonnage d'horloge variable comporte :

une unité (321) de calcul du délai de focalisation, configurée pour calculer la temporisation du signal d'écho ultrasonique réfléchi par un point focal pour arriver jusqu'à chaque élément transducteur de la barrette de transducteurs (110).

**3.** Appareil numérique de formation de faisceau, selon la revendication 2, dans lequel chacun des convertisseurs (310) analogiques-numériques (A/D) sigma-delta comporte en outre :

un convertisseur (312) A/D à un digit configuré pour émettre un signal numérique à 1 digit en réponse à une émission de l'intégrateur (311) ; et
un convertisseur (313) A/D à un digit configuré pour émettre une référence de tension en réponse à une émission du convertisseur A/D à un digit (312).

**4.** Appareil numérique de formation de faisceau (300), selon la revendication 1, dans lequel la capacité variable comporte une pluralité de capacités unitaires connectées en parallèle.

**5.** Appareil numérique de formation de faisceau (300), selon la revendication 1, dans lequel la capacité variable comporte une pluralité de capacités, chaque capacité ayant une capacitance différente déterminée selon les fréquences des signaux d'échantillonnage d'horloges.

**6.** Appareil numérique de formation de faisceau (300), selon la revendication 1, dans lequel le générateur (322) d'échantillonnage d'horloge comporte :

une unité de détection de cycle (1010) configurée pour détecter les cycles des signaux de l'échantillonnage d'horloge,
une unité de mémorisation de tableau de consultation (1020), configurée pour mémoriser les données de tableaux de consultation, relatifs à l'association des cycles des signaux de l'échantillonnage d'horloge avec l'échantillonnage des temps et des informations associées aux erreurs d'échantillonnage représentant des décalages entre les temps d'échantillonnage et les signaux d'échantillonnages d'horloges marginaux, l'unité de mémorisation de tableau de consultation étant en outre configuré pour rechercher le tableau de consultation basé sur le cycle détecté ; et
une unité de génération d'un signal de commande (1030) configurée pour émettre des signaux de commande en réponse au résultat de la recherche effectuée par l'unité (1020) de mémorisation de tableaux de consultation.

# FIG. 1
## (PRIOR ART)

# FIG. 2
## (PRIOR ART)

# FIG. 3

## FIG. 4

320

321

322

```
FOCUSING DELAY
TIME CALCULATING
UNIT
```
→
```
SAMPLING CLOCK
GENENRATOR
```
→ SAMPLING CLOCK

→ CONTROL SIGNALS

## FIG. 5

310

311

312

$x(kT)$ →
```
INTEGRATOR
```
$u(kT)$ →
```
1-bit ADC
```
$Y(kT)$ →

$q(kT)$

```
1-bit DAC
```

313

# FIG. 6

# FIG. 7

# FIG. 8

# FIG. 9

FIG. 10

322

1010

1020

1030

| CYCLE DETECTING UNIT | → | LOOKUP TABLE STORING UNIT | → | CONTROL SIGNAL OUTPUTTING UNIT |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0010638 A2
- US 5836881 A
- US 5669384 A